# EUROPÄISCHE PATENTSCHRIFT

(11) **EP 1 465 533 B1**
(45) Veröffentlichungstag und Bekanntmachung des Hinweises auf die Patenterteilung: **20.04.2011**
(21) Anmeldenummer: 03701503.9
(22) Anmeldetag: 10.01.2003
(51) Int. Cl.: A61B 17/16

(54) **CHIRURGISCHES INSTRUMENT ZUM FRÄSEN DER HÜFTPFANNE**
SURGICAL INSTRUMENT FOR GRINDING THE COTYLOID CAVITY
INSTRUMENT CHIRURGICAL DESTINE AU FRAISAGE DE LA CAVITE COTYLOIDE

(30) Priorität: 11.01.2002 WO PCT/EP02/00223; 15.05.2002 DE 10221614
(43) Veröffentlichungstag der Anmeldung: 13.10.2004
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2003/000191
(87) Internationale Veröffentlichungsnummer: WO 2003/057050

(56) Entgegenhaltungen:
- WO-A-02/102254
- US-A- 2 785 673
- US-A- 4 808 185
- US-A- 5 474 560

## Beschreibung

Zum Einsetzen einer prothetischen Pfanne in das natürliche Acetabulum muß dieses ausgefräst werden. Dazu benötigt man bislang einen in Richtung der Acetabulum-Achse offenen Zugang, um ein Fräswerkzeug mit einem entsprechend der Fräserachse verlaufenden Schaft ansetzen zu können. Dies gilt selbst bei minimal-invasiver Operationstechnik (WO 01/91648, US-A-2,785,673), wenn eine entsprechende Bohrung durch den proximalen Teil des Femurs geschaffen wird. Dies ist aufwendig und schwächt das Femur. Für Fälle schlechter Zugänglichkeit sind Fräswerkzeuge bekannt (US-A-5,176,711; US-A-4,808,185; US-B-6, 364, 910 nicht veröffentlicht), bei denen die Antriebswelle gegenüber der Drehachse des Instrumentenkopfs abgewinkelt ist. Dies erschwert die genaue Ausrichtung des Fräswerkzeugs auf die Acetabulum-Achse und die Ausübung der axialen Bearbeitungskraft. Dies gilt insbesondere bei minimal-invasivem Zugang, wenn das Operationsfeld schwer oder nicht eingesehen werden kann.

Erfindungsgemäß gelingt dies durch ein chirurgisches Instrument mit den Merkmalen des Anspruchs 1. Das Instrument weist einen Instrumentenkopf auf, der mit dem Fräser ausgerüstet wird. Der Instrumentenkopf ist mit einer Halterung verbunden, mittels der er in der gewünschten Arbeitsstellung gehalten wird. Für den Antrieb des Fräsers ist eine Antriebswelle vorgesehen, die ihn mit einer Antriebseinrichtung verbindet und die gegenüber der Drehachse des Instrumentenkopfs abgewinkelt ist. Dies gestattet es, das Instrument aus einer Richtung an das Acetabulum heranzuführen, die nicht mit der Richtung übereinstimmt, in der sich das Acetabulum öffnet. Es ist dadurch für einen minimalinvasiven Zugang zum Acetabulum geeignet, beispielsweise für einen anterolateralen oder posterolateralen Zugang.

Erfindungsgemäß ist ein Stützteil vorgesehen, der in Richtung der Drehachse auf den Instrumentenkopf wirkt und der es erlaubt, in dieser Richtung eine Bearbeitungskraft aufzubringen und/oder das Instrument auszurichten. Dieser Stützteil kann von einem Griff gebildet sein, der über einen außerhalb der Drehachse des Instrumentenkopfs liegenden Bügel mit dem Instrumentenkopf verbunden ist. Der Bügel kann auch mit einem Stift verbunden sein, der zu der Halterung des Instruments gehört. Der Bügel erlaubt es, den Instrumentenkopf von der Seite her durch eine gegebenenfalls enge Operationsöffnung an das Acetabulum heranzubringen und dann noch in der Richtung der Arbeitsachse eine Vorschubkraft auszuüben. Auch gewinnt der Operateur dadurch eine genaue Vorstellung davon, wie die Bearbeitungsachse des Instruments liegt, was ihm die Ausrichtung des Instruments erleichtert.

Die Abstützung des Instrumentenkopfs kann bei einer anderen Ausführungsform der Erfindung auch am Femur erfolgen, der insbesondere bei minimal-invasiver Operationstechnik hinreichend durch Bänder und Muskeln mit dem Hüftknochen verbunden ist und daher in der Lage ist, die Bearbeitungskräfte aufzunehmen. Ferner kann nach der Erfindung der Femurhals zur Ausrichtung des Instruments benutzt werden. Dies beruht auf dem Gedanken, daß die Richtung des Femurhalses in vielen Fällen mit der gewünschten Richtung des Fräsers übereinstimmt. Jedenfalls gilt dies dann, wenn der Bandapparat genügend erhalten ist und der Operateur dafür sorgt, daß das Bein eine natürliche Lage gegenüber dem Acetabulum einnimmt. Für diese Abstützung am Femur kann die Halterung des Instrumentenkopfs einen auf der dem Fräser gegenüberliegendem Seite des Instrumentenkopfs angeordneten Femur-Ansatzteil aufweisen. Dieser kann - je nach den gewünschten Zwecken - nur abstützend oder nur richtungsbestimmend oder beides sein. Auf Einzelheiten wird weiter unten näher eingegangen.

Die Halterung des Instrumentenkopfs umfaßt zweckmäßigerweise einen ebenso wie die Antriebswelle gegenüber der Drehachse des Instrumentenkopfs abgewinkelten Schaft. Die Abwinklung des Schafts bzw. der Antriebswelle gegenüber der Drehachse des Instrumentenkopfs liegt zweckmäßigerweise zwischen 90 und 135°, gemessen auf der dem Fräser zugewandten Seite. Dieser Winkel kann - wie bei den genannten bekannten Instrumenten - unveränderbar sein. Er kann aber auch verstellbar und ggf. in einer gewünschten Position fixierbar oder auch frei variabel sein, so daß der Arzt die Richtung des Griffs während des Fräsens den jeweiligen Verhältnissen optimal anpassen kann. Die Antriebswelle kann mit dem Schaft verbunden sein. Jedoch ist dies nicht unbedingt erforderlich. Sie ist vorzugsweise flexibel, wenn kein Schaft vorgesehen ist oder wenn er gesondert von der Antriebswelle ist.

Im allgemeinen ist der Schaft bleibend mit dem Instrumentenkopf verbunden. Jedoch kann es mitunter vorteilhaft sein, wenn er leicht lösbar ist, um von Fall zu Fall, ggf. auch intraoperativ, mit dem Instrumentenkopf verbunden bzw. von ihm gelöst werden zu können. Man kann dann auch unterschiedlich gestaltete oder gerichtete Schäfte vorsehen, um unterschiedlichen Vorstellungen des Operateurs und unterschiedlichen anatomischen Verhältnissen gerecht werden zu können. Das gilt insbesondere dann, wenn der weiter oben erwähnte Bügel mit dem Schaft verbunden ist. Der Schaft ist in der lösbaren Ausführung nicht mit der Antriebswelle verbunden.

Wenn zur Abstützung des Instrumentenkopfes und/oder zur Bestimmung seiner Richtung das Femur verwendet wird, kann dies mittels eines Ansatzteils erfolgen, der den Femurhals auf einer gewissen Länge außen umfaßt. Bei einer anderen, bevorzugten Ausführung der Erfindung wird der Ansatzteil von einem in Richtung der Drehachse des Fräsers verlaufenden Zapfen gebildet, der passend mit einer Bohrung zusammenwirkt, die der Operateur zuvor von der Resektionsfläche her in den Femurhals eingebracht hat. Dieser Zapfen kann mit einem Gehäuseteil des Fräsers feststehend verbunden sein. Er kann auch von einer Fortsetzung der Fräserwelle gebildet sein und sich mit dem Fräser drehen. In diesem Fall ist es zweckmäßig, wenn eine in die Bohrung des Femurhalses einsetzbare Laufbuchse zur Aufnahme des Zapfens vorgesehen ist. Der Femuransatzteil kann auch mit Mitteln versehen sein, die den Instrumentenkopf gegen Drehung sichern.

Der Femuransatzteil kann die einzige Halterung für den Instrumentenkopf bilden. Jedoch ist es oft zweckmäßig, zusätzlich den erwähnten Schaft vorzusehen, damit die Ausrichtung des Instruments und die Vorschuberzeugung von außen kontrolliert und beeinflußt werden kann. In diesem Anwendungsfall kann die oben erwähnte Lösbarkeit zweckmäßig sein. Ferner kann es zweckmäßig sein, den Femuransatzteil und den Schaft so auszubilden, daß sie alternativ eingesetzt werden können. Beispielsweise kann der am Instrumentenkopf dem Femur zugewandte Zapfen alternativ zum Verbinden mit dem Schaft statt mit dem Femur dienen. Gewünschtenfalls kann die Verbindung zwischen dem Instrumentenkopf und dem Schaft bzw. dem Femur so ausgebildet sein, daß über den Schaft bzw. das Femur das Fräsmoment aufgenommen wird.

Bei derjenigen Ausführung, bei der das Femur als Gegenlager bei der Erzeugung der Vorschubkraft eingesetzt wird, kann die Vorschubkraft dadurch erzeugt oder erhöht werden, daß der Femuransatzteil eine Einrichtung zum Abstützen am Femur umfaßt und daß zwischen dieser Abstützeinrichtung und dem Instrumentenkopf ein Streckmechanismus angeordnet ist. Dieser Streckmechanismus drückt den Instrumentenkopf von der Abstützeinrichtung weg, wenn ein entsprechender Vorschubantrieb auf ihn ausgeübt wird. Er kann zu diesem Zweck mit einer Vorschubantriebsstange fest oder lösbar verbunden sein. Diese soll gegenüber der Drehachse des Instrumentenkopfs ebenso wie die Antriebswelle und/oder der Schaft abgewinkelt sein, und zwar etwa zu derselben Seite hin. Der Streckmechanismus bildet ein Getriebe in seinem allgemeinsten Sinne, das die von der Vorschubantriebsstange vorgegebene Bewegung in eine Streckung des Abstands zwischen dem Instrumentenkopf und der Abstützeinrichtung umwandelt. Dem Techniker stehen unterschiedlichste Getriebeausführungen zu diesem Zweck ohne weiteres zur Verfügung. Beispielsweise kann ein Hebelgetriebe gewählt werden, das eine Schwenkbewegung der Vorschubantriebsstange um eine quer zur Drehachse des Instrumentenkopfs liegende Schwenkachse in eine Streckbewegung des Streckmechanismus umwandelt. Dabei kann der Streckmechanismus mit der Vorschubantriebsstange als Spreizzange ausgebildet sein; in diesem Fall wird die Vorschubantriebsstange von einem Zangenhebelpaar gebildet. Der Streckmechanismus kann auch von einem Drehgetriebe gebildet sein; in diesem Fall wird die Vorschubantriebsstange gedreht, um beispielsweise über ein an ihrem Ende vorgesehenes Ritzel auf eine Zahnstange zu wirken, die die Streckung des Mechanismus bewirkt.

Damit der Instrumentenkopf von der Seite her durch eine enge Operationsöffnung herangeführt werden kann, soll seine Abmessung in Richtung der Fräserachse möglichst gering sein. Ein Maß für diese Abmessung ist der Abstand zwischen dem Kreuzungspunkt der Drehachse des Instrumentenkopfs mit der Achse des Schafts oder der Antriebswelle einerseits und dem Mittelpunkt des Fräsers andererseits. Dieser Abstand soll möglichst gering sein. Er soll nicht größer sein als der Fräserdurchmesser und vorzugsweise nicht größer als der halbe Fräserdurchmesser. Die in Richtung der Fräserachse gemessene Außenabmessung des Instrumentenkopfs ist zweckmäßigerweise nicht größer als 8 cm, vorzugsweise nicht größer als 6 cm.

Die Erfindung wird im folgenden näher unter Bezugnahme auf die Zeichnung erläutert, die vorteilhafte Ausführungsbeispiele veranschaulicht. Es zeigen:
- Fig. 1: einen Längsschnitt durch das Instrument,
- Fig. 2: die schematische Ansicht einer Ausführungs- alternative des Instruments,
- Fig. 3: eine dritte Ausführungsform des Geräts im Einsatz,
- Fig. 4: eine vierte Ausführungsform und
- Fig. 5: eine fünfte Ausführungsform.

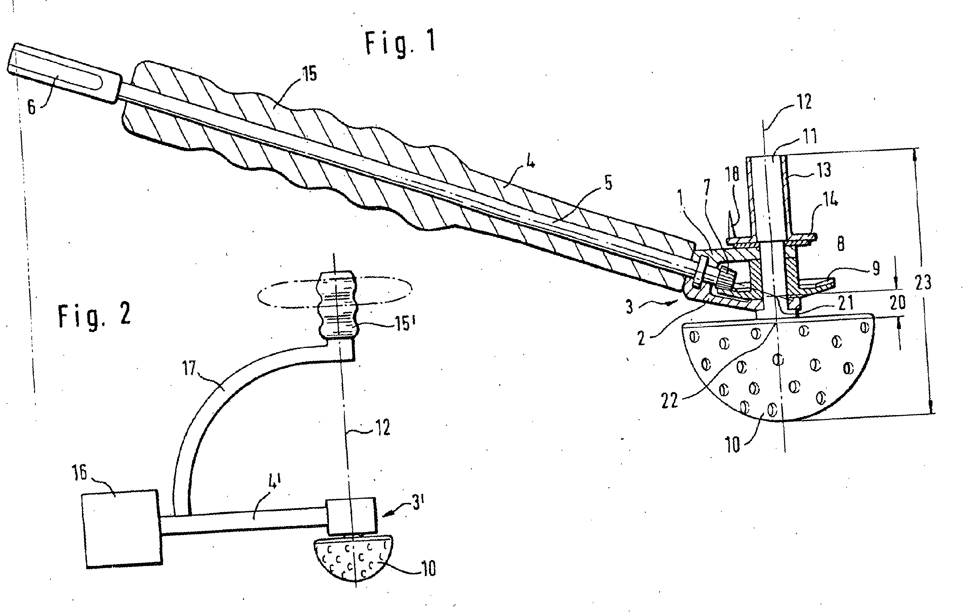

Die Gehäuseteile 1, 2 des in Fig. 1 gezeigten Instrumentenkopfs 3 sind mit einem Schaft 4 fest verbunden, der einen Handgriff 15 bildet und eine Antriebswelle 5 enthält, deren freies Ende 6 in bekannter Weise mit einem Antriebsmotor oder einem Griff für Handantrieb verbunden werden kann. Innerhalb des Gehäuses 1, 2 trägt die Antriebswelle 5 ein Zahnritzel 7.

In dem Gehäuse 1, 2 des Instrumentenkopfs 3 ist ferner eine Fräserwelle 8 drehbar quer zu der Antriebswelle 5 gelagert. Der Winkel zwischen den beiden Wellen liegt zweckmäßigerweise zwischen 90 und 135° (im dargestellten Fall 110° ). Die Fräserwelle trägt ein Kegelrad 9, dessen Zähne mit denen des Ritzels 7 in Eingriff stehen. Die Fräserwelle 8 läßt sich auf diese Weise durch die Antriebswelle 5 drehend antreiben. Es versteht sich, daß dies nur ein Beispiel für die Antriebsverbindung zwischen den beiden Wellen darstellt. Es können auch andere Getriebearten, beispielsweise ein Schneckengetriebe, verwendet werden. Das Getriebe selbst oder ein damit verbundener Kardanteil kann winkeltolerant ausgebildet sein, um eine verstellung des Winkels zwischen den Wellen 5 und 8 zu ermöglichen. Es kann eine Fixiereinrichtung vorgesehen sein, die es gestattet, das Instrument in der gewählten Winkeleinstellung zu arretieren. Stattdessen kann auch eine freie Winkelbeweglichkeit vorgesehen sein.

Auf das Ende der Fräserwelle 8 ist in bekannter Weise auswechselbar ein halbkugeliges, als solches bekanntes Fräswerkzeug 10 aufgesetzt, das in dieser Beschreibung einfachheitshalber als Fräser bezeichnet wird. Um die Abmessung des Instrumentenkopfs gering zu halten, ist der Fräser eng an das Gehäuse 1, 2 des Instrumentenkopfs herangerückt. Die Entfernung des Kugelmittelpunkts 22 des Fräsers oder des Mittelpunkts seiner Rückfläche von dem Kreuzungspunkt 21 der Achsen der Wellen 5 und 8 ist kleiner als der Halbmesser des Fräsers, vorzugsweise kleiner als ein Viertel des Fräserdurchmessers. Wenn der Fräser nicht halbkugelig geformt ist, tritt an die Stelle des erwähnten Durchmessers der größte Durchmesser.

Auf der dem Fräser 10 abgewandten Seite des Instrumentenkopfs 3 ragt ein Zapfen 11 hervor, der fest, ggf. einstückig, mit der Fräserwelle 8 verbunden ist und mit dieser eine gemeinsame Drehachse 12 hat. Eine Hülse 13 mit Endflansch 14 ist auf den Zapfen 11 mit Gleitsitz aufgesteckt. Der Flansch trägt auf seiner dem Knochen zugewendeten Seite Zähne oder Nadeln 18, die in den Knochen eindringen, um die Drehung der Hülse 13 zu verhindern.

Das Instrument wird in folgender Weise benutzt. Nachdem der Hüftkopf entfernt ist, wird in den Femurhals von dessen Resekionsfläche her achsgleich eine Sackbohrung eingebracht, in die die Hülse 13 eingesteckt wird. Das Instrument wird so eingebracht, daß der Zapfen 11 in die in der Femurbohrung sitzende Hülse 13 gesteckt wird. Das Bein wird normal gelagert, so daß der Femurhals zum Acetabulum weist und der Fräser anstelle des natürlichen Hüftkopfs im oder am Acetabulum liegt. Der Fräser 10 wird durch die den proximalen Femur haltenden Bänder in das Acetabulum gedrückt und gleichzeitig in natürlicher Weise ausgerichtet. Das Ausfräsen des Acetabulums kann nun ohne oder mit geringer ausrichtender Hilfe des Operateurs vollzogen werden. Danach wird die Operation in bekannter Weise fortgeführt.

Während das Ausführungsbeispiel gemäß Fig. 1 sich mit einem quer vom Instrumentenkopf 3 abstehenden Griffschaft begnügt, ist in Fig. 2 schematisch ein Instrument angedeutet, dessen Griff 15' etwa in der Achse 12 des Fräsers 10 angeordnet ist. Der Schaft 4' des Instruments führt etwa lotrecht vom Instrumentenkopf 3' weg und trägt am Ende einen Antriebsmotor 16. Durch einen Bügel 17 ist das Ende des Schafts 4' starr mit dem Griff 15' verbunden. Dessen Lage zeigt dem Operateur genau die Stellung der Fräserachse 12 an und erlaubt es ihm, nicht nur den Fräser gemäß der gewünschten Achsrichtung auszurichten, sondern auch die Kraft zu steuern, mit der der Fräser in das Acetabulum gedrückt wird. Der Griff kann in der strichpunktierten Linie flach ausgebildet sein, um die Kraftausrichtung mit der Hand oder dem Körper des Operateurs zu erleichtern.

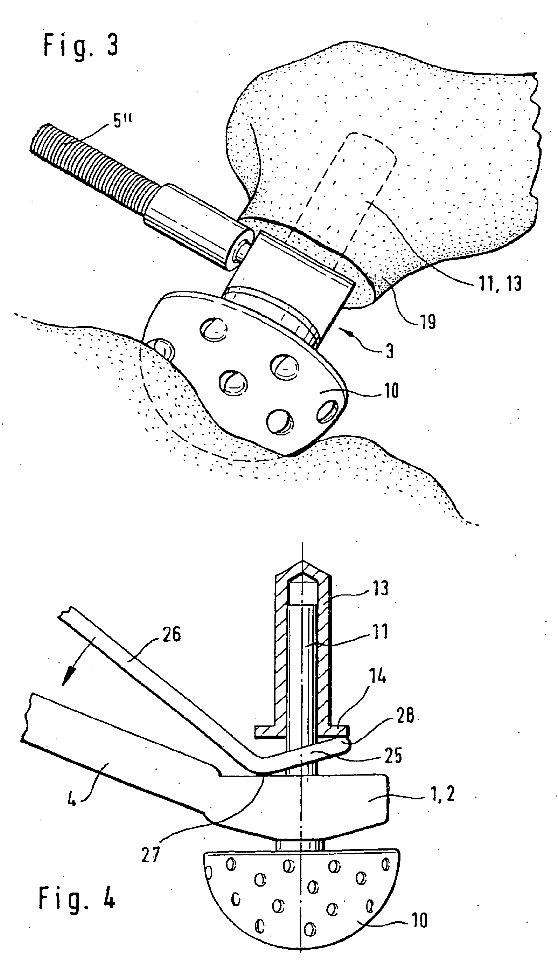

Das Instrument gemäß Fig. 3 unterscheidet sich von dem in Fig. 1 gezeigten dadurch, daß ihm ein Schaft fehlt und die Antriebswelle 5" als flexibles Welle ausgebildet ist. Die Ausrichtung des Instruments wird - wie unter Bezugnahme auf Fig. 1 erläutert - durch die in den Knochen 19 eingesetzte Hülse 13 und den darin gelagerten Zapfen 11 bewirkt. Auch die zum Fräsen erforderliche Vorschubkraft wird vom Knochen 19 her auf das Instrument übertragen. Die durch das Femur bestimmte Ausrichtung des Instruments wird dank der Flexibilität der Antriebswelle 5' nicht durch unbeabsichtigte Bewegungen des Operateurs beeinträchtigt. Jedoch ist die Welle 2' so steif, daß sie das Fräsmoment zu übertragen vermag. Es kann auch eine drehfeste Verbindung zwischen dem Instrumentenkopf 3 und dem Flansch der Hülse 13 vorgesehen sein, wobei der Flansch wiederum durch Zähne oder Nadeln 18 (Fig. 1) drehfest mit dem Knochen verbunden ist, um sich in bezug auf das Fräsmoment an diesem abzustützen.

In Fig. 4 ist eine Variante des Instruments gezeigt, für dessen Beschreibung auf Fig. 1 verwiesen werden kann. Zwischen dem Gehäuse 1, 2 und dem Flansch 14 ist das Ende 25 eines Hebels 26 eingeschaltet. Dieses Ende ist gabelig ausgeführt, um gewünschtenfalls nachträglich zwischen das Gehäuse 1, 2 und den Flansch 14 eingeschoben werden zu können. Es ist gegenüber dem langen Ende 26 des Hebels ein wenig abgewinkelt. Es wird zunächst parallel zur Rückfläche des Gehäuses 1, 2 und zum Flansch 14 zwischen diesen beiden Teilen eingeschoben. Das lange Ende 26 des Hebels befindet sich dann in einer gewissen Winkelentfernung von dem Schaft 4. Wenn eine Vorschubkraft erzeugt werden soll, die den Fräser 10 in das Acetabulum drückt, wird das lange Ende 26 des Hebels in Pfeilrichtung zum Schaft 4 hin gedrückt. Diese Bewegung kann der Operateur leicht in der Art der Betätigung einer Zange durchführen. Dabei setzt sich der Knickpunkt 27 des Hebels auf die Rückfläche des Gehäuses 1, 2 auf und bildet den Drehpunkt für seine anschließende Schwenkbewegung. Die Spitze 28 setzt sich auf den Flansch 14 auf und drückt ihn bei fortdauernder Schwenkbewegung von dem Gehäuse 1, 2 weg. Das Hebelende 25 bildet somit gemeinsam mit der Rückfläche des Gehäuses 1, 2 und dem Flansch 14 einen Streckmechanismus. Das lange Ende 26 des Hebels bildet eine Vorschubantriebsstange, durch deren Bewegung der Streckmechanismus gestreckt wird, um die Distanz des Fräsers 10 von dem Knochen 19, an dem sich der Flansch 14 der Hülse 13 abstützt, zu vergrößern und so den Fräser 11 in das Acetabulum zu drücken.

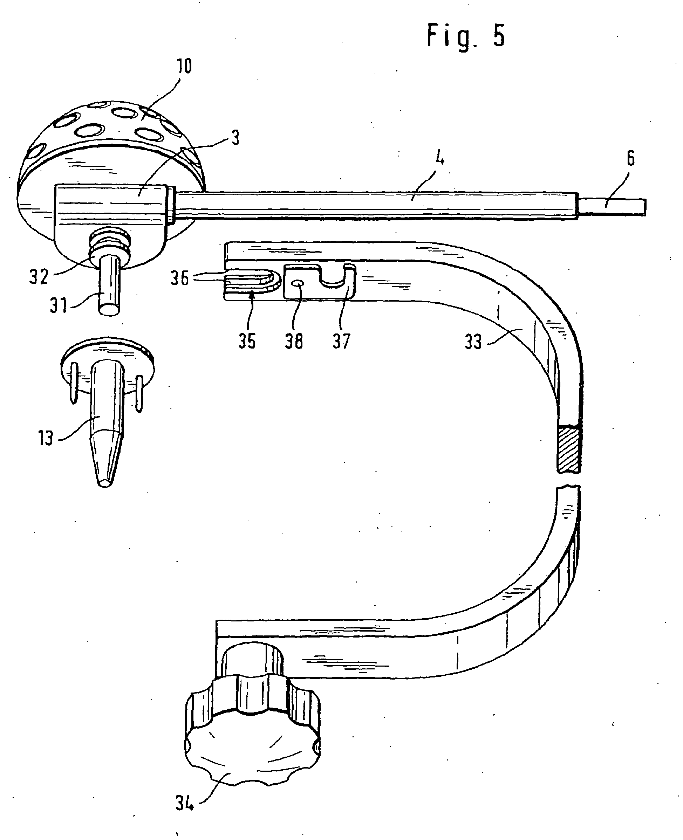

In der fünften Ausführungsform gemäß Fig. 5 ist mit dem Instrumentenkopf 3 in der oben erläuterten Weise ein Fräser 10 verbunden. Rechtwinklig zur Fräserachse ist mit dem Instrumentenkopf 3 ein Schaft 4 verbunden, der eine Antriebswelle enthält, mit deren freien Ende 6 ein Antriebsmotor gekuppelt werden kann. Mit dem Instrumentenkopf 3 ist ein Zapfen 31 starr verbunden. Er kann in die Bohrung einer Hülse 13 eingesetzt werden, die ihrerseits in den nach Resektion des Hüftkopfs verbleibenden Schenkelhals eingesetzt ist, wie es oben beschrieben wurde. Der Zapfen 31 dient dann zur Ausrichtung des Instrumentenkopfs 3 und des Fräsers 10 gemäß der Richtung des Schenkelhalses des Oberschenkelknochens. Auch kann dadurch die erforderliche Andruckkraft auf den Fräser 10 übertragen werden.

Um zusätzliche Kraft auf den Instrumentenkopf 3 übertragen zu können und gegebenenfalls auch das Instrument ausrichten zu können, ist ein Bügel 33 mit Griff 34 vorgesehen. Der Griff liegt in derselben Achse wie die Drehachse des Fräsers 10 und die Achse des Zapfens 31. Der Bügel ist hinreichend steif ausgebildet, um die Andruck- und Führungskraft von dem Griff 34 auf den Instrumentenkopf 3 übertragen zu können. Am Instrumentenkopf 3 und am griffernen Ende des Bügels 33 sind zueinander passende Kupplungsorgane 32, 35 vorgesehen, die vorzugsweise so ausgebildet sind, daß sie rasch und einfach geschlossen oder gelöst werden können, wie es während der Operation notwendig werden kann. Die Technik stellt für derartige Kupplungen eine Vielzahl von Ausführungsmöglichkeiten zur Verfügung. Im dargestellten Beispiel sind sie folgendermaßen ausgebildet. Am Bügel ist eine Kupplungsgabel 35 vorgesehen, die eine Gabelöffnung enthält, welche von profilierten Gabelflanken 36 beiderseits begrenzt wird. Das Kupplungselement 32 am Instrumentenkopf 3 ist passend zur Gabelöffnung ausgebildet und weist eine Profilierung auf, die zu der Profilierung der Flanken 36 komplementär ist. Beispielsweise können die Flanken 36 eine mittlere Rippe enthalten, der an dem Kupplungselement 32 eine Nut entspricht. Das Kupplungselement 32 ist rund ausgebildet, so daß die Kupplungsgabel 35 von jeder Seite her aufgesteckt werden kann. Diese Konstruktion gibt dem Operateur Freiheit bezüglich der Richtung, von der her er den Bügel an das Instrument ansetzen will. Wenn dies nicht gewünscht wird, kann das Kupplungselement 32 beispielsweise vierkantig mit parallelen profilierten Seiten ausgebildet sein, die in die Kupplungsöffnung der Gabel 35 passen, aber mit dieser eine Kupplungsrichtung bestimmen.

Damit die Kupplungsgabel 35 nicht ungewollt von dem instrumentenseitigen Kupplungselement 32 abgleiten kann, ist ein Sicherungshaken 37 vorgesehen, der um eine Achse 38 schwenkbar ist, um aus der Lösestellung, die in Fig. 5 gezeigt ist, in die Sicherungsstellung überführt werden zu können, in der sie um den Zapfen 31 faßt, wenn das Kupplungselement 32 sich in der Gabelöffnung befindet, und umgekehrt. Der Sicherungshaken kann mit einer Rasteinrichtung verbunden sein, die seine ungewollte Entfernung aus der Sicherungsposition verhütet.

## Patentansprüche

1. Chirurgisches Instrument, insbesondere zum Fräsen der Hüftpfanne, das einen Instrumentenkopf (3) mit Fräser (10), eine Halterung zum Halten des Instrumentenkopfs (3) in der Arbeitsstellung und eine den Fräser (10) mit einer Antriebseinrichtung (16) verbindende Antriebswelle (5, 5") umfasst, die gegenüber der Drehachse (12) des Instrumentenkopfs (3, 3') abgewinkelt ist, **dadurch gekennzeichnet, dass** ein in Richtung der Drehachse (12) auf den Instrumentenkopf (3') wirkender Stützteil mit einem Griff (15') vorgesehen ist, der über einen außerhalb der Drehachse (12) liegenden Bügel (17) der Halterung mit dem Instrumentenkopf (3') oder einem Schaft (4') der Halterung, der entsprechend der Antriebswelle (5, 5") gegenüber der Drehachse (12) des Instrumentenkopfs (3, 3') abgewinkelt ist, verbunden ist.

2. Instrument nach Anspruch 1, **dadurch gekennzeichnet, dass** die Antriebswelle (5) in oder an dem Schaft (4, 4") angeordnet ist.

3. Instrument nach einem der Ansprüche 1 bis 2, **dadurch gekennzeichnet, dass** der Stützteil von einem auf der dem Fräser (10) gegenüberliegenden Seite des Instrumentenkopfs (3) angeordneten Femur-Ansatzteil (11, 13) gebildet ist.

4. Instrument nach Anspruche 3, **dadurch gekennzeichnet, daß** der Femur-Ansatzteil (11, 13) richtungsbestimmend ist.

5. Instrument nach Anspruch 4, **dadurch gekennzeichnet, daß** der Femur-Ansatzteil einen in Richtung der Drehachse (12) des Instrumentenkopfs (3) verlaufenden Zapfen (11) umfasst.

6. Instrument nach Anspruch 5, **dadurch gekennzeichnet, dass** der Zapfen (11) von der Fräserwelle (8) gebildet ist.

7. Instrument nach Anspruch 6, **dadurch gekennzeichnet, dass** eine in eine Bohrung im Femur einsetzbare Laufbuchse (13) für den von der Fräserwelle (8) gebildeten Zapfen (11) vorgesehen ist.

8. Instrument nach einem der Ansprüche 1 bis 7, **dadurch gekennzeichnet, dass** der Winkel zwischen der Drehachse (12) des Instrumentenkopfs (3) und der Antriebswelle (5) und/oder dem Schaft (4) verstellbar ist.

9. Instrument nach einem der Ansprüche 1 bis 8, **dadurch gekennzeichnet, dass** ein gesonderter Schaft (26) leicht lösbar mit dem Instrumentenkopf (3) verbindbar ist.

10. Instrument nach einem der Ansprüche 1 bis 9, **dadurch gekennzeichnet, dass** eine Fräser-Vorschubeinrichtung zum Vorschieben des Instrumentenkopfs (3) unter Abstützung am Femur vorgesehen ist.

11. Instrument nach Anspruch 10, **dadurch gekennzeichnet, dass** der Femur-Ansatzteil (11, 13) eine Einrichtung (14) zum Abstützen am Femur umfasst und dass zwischen dieser Abstützeinrichtung (14) und dem Instrumentenkopf (3) ein Streckmechanismus (25) angeordnet ist.

12. Instrument nach einem der Ansprüche 1 bis 11, **dadurch gekennzeichnet, dass** der Abstand (20) zwischen dem Kreuzungspunkt (21) der Drehachse (12) des Instrumentenkopfs (3) mit der Achse des Schafts (4) oder der Antriebswelle (5) einerseits und dem Mittelpunkt (22) des Fräsers (10) andererseits nicht größer als der Fräserdurchmesser ist.

13. Instrument nach Anspruch 12, **dadurch gekennzeichnet, dass** der Abstand (20) nicht größer als der halbe Fräserdurchmesser ist.

14. Instrument nach einem der Ansprüche 1 bis 13, **dadurch gekennzeichnet, dass** die in Richtung der Fräserachse (12) gemessene Außenabmessung (23) des Instruments ohne seine gegebenenfalls im Femur unterzubringenden Teile einschließlich des Fräsers (10) nicht größer als 8 cm ist.

## Claims

1. Surgical instrument, in particular for grinding the cotyloid cavity, comprising an instrument head (3) with grinder (10), a holder for holding the instrument head (3) in the working position, and a drive shaft (5, 5") which connects the grinder (10) to a drive device (16) and is located at an angle with respect to the rotation axis (12) of the instrument head (3, 3'), **characterized in that** a support part having a handle (15'), arranged in the direction of the rotation axis (12) and acting on the instrument head (3') is provided, which support part is connected to the instrument head (3') or to a shank (4') of the holder, which shank is located, like the drive shaft (5, 5"), at an angle with respect to the rotation axis (12) of the instrument head (3, 3'), via a bow (17) of the holder located outside of the rotation axis (12).

2. Instrument according to Claim 1, **characterized in that** the drive shaft (5) is arranged in or on the shank (4, 4").

3. Instrument according to either of Claims 1 and 2, **characterized in that** the support part is formed by a femur attachment part (11, 13) arranged on that side of the instrument head (3) facing away from the grinder (10).

4. Instrument according to Claim 3, **characterized in that** the femur attachment part (11, 13) determines direction.

5. Instrument according to Claim 4, **characterized in that** the femur attachment part comprises a journal (11) extending in the direction of the rotation axis (12) of the instrument head (3).

6. Instrument according to Claim 5, **characterized in that** the journal (11) is formed by the grinder shaft (8).

7. Instrument according to Claim 6, **characterized in that** a bushing (13) which can be inserted into a bore in the femur is provided for the journal (11) formed by the grinder shaft (8).

8. Instrument according to one of Claims 1 to 7, **characterized in that** the angle between the rotation axis (12) of the instrument head (3) and the drive shaft (5) and/or the shank (4) is adjustable.

9. Instrument according to one of Claims 1 to 8, **characterized in that** a separate shank (26) can be connected to the instrument head (3) in an easily detachable manner.

10. Instrument according to one of Claims 1 to 8, **characterized in that** a grinder advancer device is provided for advancing the instrument head (3) with support on the femur.

11. Instrument according to Claim 10, **characterized in that** the femur attachment part (11, 13) comprises a means (14) for support on the femur, and **in that** an extension mechanism (25) is arranged between this support means (14) and the instrument head (3) .

12. Instrument according to one of Claims 1 to 11, **characterized in that** the distance (20) between the intersection point (21) of the rotation axis (12) of the instrument head (3) and the axis of the shank (4) or drive shaft (5), on the one hand, and the center point (22) of the grinder (10), on the other hand, is not greater than the grinder diameter.

13. Instrument according to Claim 12, **characterized in that** the distance (20) is not greater than half the grinder diameter.

14. Instrument according to one of Claims 1 to 13, **characterized in that** the outer dimension (23) of the instrument, measured in the direction of the grinder axis (12), without its parts optionally to be accommodated in the femur, and including the grinder (10), is not greater than 8 cm.

## Revendications

1. Instrument chirurgical, notamment pour le fraisage de la cavité cotyloïde, qui comprend une tête d'instrument (3) avec une fraise (10), une fixation pour fixer la tête d'instrument (3) dans la position de travail et un arbre d'entraînement (5, 5") reliant la fraise (10) à un dispositif d'entraînement (16), lequel est coudé par rapport à l'axe de rotation (12) de la tête d'instrument (3, 3'), **caractérisé en ce qu'**une partie de support agissant dans la direction de l'axe de rotation (12) sur la tête d'instrument (3') est pourvue d'une poignée (15') qui est connectée, par le biais d'une anse (17) de la fixation située en dehors de l'axe de rotation (12), à la tête d'instrument (3') ou à une tige (4') de la fixation, qui est coudée de manière correspondant à l'arbre d'entraînement (5, 5") par rapport à l'axe de rotation (12) de la tête d'instrument (3, 3').

2. Instrument selon la revendication 1, **caractérisé en ce que** l'arbre d'entraînement (5) est disposé dans ou sur la tige (4, 4").

3. Instrument selon l'une quelconque des revendications 1 à 2, **caractérisé en ce que** la partie de support est formée par un appendice pour le fémur (11, 13) disposé du côté de la tête d'instrument (3) opposé à la fraise (10).

4. Instrument selon la revendication 3, **caractérisé en ce que** l'appendice pour le fémur (11, 13) détermine l'orientation.

5. Instrument selon la revendication 4, **caractérisé en ce que** l'appendice pour le fémur comprend un tourillon (11) s'étendant dans la direction de l'axe de rotation (12) de la tête d'instrument (3).

6. Instrument selon la revendication 5, **caractérisé en ce que** le tourillon (11) est formé par l'arbre de la fraise (8).

7. Instrument selon la revendication 6, **caractérisé en ce qu'**il est prévu une douille de glissement (13) pouvant être insérée dans un alésage du fémur pour le tourillon (11) formé par l'arbre de la fraise (8).

8. Instrument selon l'une quelconque des revendications 1 à 7, **caractérisé en ce que** l'angle entre l'axe de rotation (12) de la tête d'instrument (3) et l'arbre d'entraînement (5) et/ou la tige (4) est réglable.

9. Instrument selon l'une quelconque des revendications 1 à 8, **caractérisé en ce qu'**une tige séparée (26) peut être raccordée, de manière facilement détachable, à la tête d'instrument (3).

10. Instrument selon l'une quelconque des revendications 1 à 9, **caractérisé en ce qu'**un dispositif d'avance de fraise est prévu pour faire avancer la tête d'instrument (3) en s'appuyant contre le fémur.

11. Instrument selon la revendication 10, **caractérisé en ce que** l'appendice pour le fémur (11, 13) comprend un dispositif (14) pour s'appuyer contre le fémur et **en ce qu'**entre ce dispositif d'appui (14) et la tête d'instrument (3) est disposé un mécanisme d'étirement (25).

12. Instrument selon l'une quelconque des revendications 1 à 11, **caractérisé en ce que** la distance (20) entre le point d'intersection (21) de l'axe de rotation (12) de la tête d'instrument (3) avec l'axe de la tige (4) ou de l'arbre d'entraînement (5) d'une part et le centre (22) de la fraise (10) d'autre part, n'est pas supérieure au diamètre de la fraise.

13. Instrument selon la revendication 12, **caractérisé en ce que** la distance (20) n'est pas supérieure à la moitié du diamètre de la fraise.

14. Instrument selon l'une quelconque des revendications 1 à 13, **caractérisé en ce que** la dimension extérieure (23) de l'instrument mesurée dans la direction de l'axe de la fraise (12), sans ses pièces devant éventuellement être insérées dans le fémur et y compris la fraise (10), n'est pas supérieure à 8 cm.
